# EUROPEAN PATENT APPLICATION

(11) **EP 3 076 318 A1**
(43) Date of publication of application: **05.10.2016**
(21) Application number: 16152039.0
(22) Date of filing: 20.01.2016
(51) Int. Cl.: G06F 19/00

(54) **DATA FORMAT FOR CLINICAL TEST CREATION SUPPORTING PROGRAM, DATA FORMAT FOR CLINICAL TEST CREATION SUPPORTING METHOD, AND INFORMATION PROCESSING DEVICE**

(30) Priority: 31.03.2015 JP 2015072013
(71) Applicant: FUJITSU LIMITED, Kawasaki-shi, Kanagawa 211-8588 (JP)
(72) Inventor: Ebi, Kunihito, Kanagawa, 211-8588 (JP); Yamakawa, Kazuo, Osaka, 540-8514 (JP); Okuyama, Chiaki, Kanagawa, 211-8588 (JP)
(74) Representative: Hoffmann Eitle

(57) **Abstract**

A data format for clinical test creation supporting program causes a computer to execute process including, accepting (131) registration of a name of a data item used in a clinical test; searching (132) a first name associated with a second name corresponding to the accepted name of the data item with reference to a storage unit that stores the second name in association with the first name related to the data item used in the clinical test; and outputting (133) a search result screen on which the first name searched from the storage unit is displayed.

## Description

### FIELD

The embodiments discussed herein are related to a data format for clinical test creation supporting program, a data format for clinical test creation supporting method, and an information processing device.

### BACKGROUND

Conventionally, in a clinical test of drugs and the like, case data is collected as a paper-based CRF (case report form) in a medical institution. Recently, the case data is collected by an EDC (electronic data capture) system by using an electronic case report form (eCRF). The case data collected by the EDC system is managed by a CDMS (clinical data management system) system. A pharmaceutical company and the like generates clinical test data to be submitted to a regulatory authority based on the case data managed by the CDMS system, for example.

A CDISC (Clinical Data Interchange Standards Consortium)-compliant format is used, for example, for the clinical test data to be submitted. The CDISC-compliant format includes a SDTM (Study Data Tabulation Model) for clinical data and an ADaM (Analysis Data Model) for statistical analysis data, for example. Therefore, the clinical test data is converted from an EDC/CDMS system format to a CDISC format.

A system which transfers data between databases is proposed. The system reads a plurality of transfer source item names from a contract result database which stores transfer source data to be transferred in association with the transfer source item name and accepts the transfer when transferring the data between the databases. When the system accepts the transfer, this reads a transfer destination item name from a database for analysis which stores in association with the transfer destination item name to calculate matching degrees between a plurality of transfer source item names and the transfer destination item name. The system outputs the transfer source item name the matching degree of which is calculated to be the highest in association with the transfer destination item name as a candidate of the transfer source item name corresponding to the transfer destination item name to which the transfer source data is to be transferred.

When a data item name in a program belongs to a plurality of data definition objects and an old name of the data item corresponds to a plurality of new names, it is proposed that the old name of the data item is converted to the new name based on an old name-new name correspondence table and presence or absence of a modification instruction indicating a higher-order layer of the data item name.
[Patent Literature 1] Japanese Laid-open Patent Publication No. 2004-259209
[Patent Literature 2] Japanese Laid-open Patent Publication No. 7-152545

However, there is a case in which a rule of the data item, that is to say, a data format is not pervasive in an organization such as the medical institution and the pharmaceutical company which perform a series of clinical tests. Therefore, if the data item in the EDC/CDMS system format is the data item which is not defined in the CDISC, this is converted to a different data item by a person in charge of the organization performing the conversion, so that management of the clinical test becomes difficult.

Accordingly, it is an object in one aspect of an embodiment of the invention to provide a data format for clinical test creation supporting program, a data format for clinical test creation supporting method, and an information processing device capable of efficiently creating the data format.

### SUMMARY

According to an aspect of an embodiment, a data format for clinical test creation supporting program causes a computer to execute a process including, accepting (131) registration of a name of a data item used in a clinical test; searching (132) a first name associated with a second name corresponding to the accepted name of the data item with reference to a storage unit that stores the second name in association with the first name related to the data item used in the clinical test; and outputting (133) a search result screen on which the first name searched from the storage unit is displayed.

### BRIEF DESCRIPTION OF DRAWINGS

FIG. 1 is a block diagram illustrating an example of a configuration of an information processing system of a first embodiment;
FIG. 2 is a view illustrating an example of a dictionary storage unit;
FIG. 3 is a view illustrating an example of a definition for collection storage unit;
FIG. 4 is a view illustrating an example of a definition for statistics storage unit;
FIG. 5 is a view illustrating an example of a doctor input screen;
FIG. 6 is a view illustrating an example of an assignment screen;
FIG. 7 is a view illustrating an example of a search result screen;
FIG. 8 is a view illustrating another example of the search result screen;
FIG. 9 is a view illustrating an example of an assignment result screen;
FIG. 10 is a view illustrating an example of a case in which definition is generated from actual data of the first embodiment;
FIG. 11 is a flowchart illustrating an example of a definition generating process of the first embodiment;
FIG. 12 is a view illustrating an example of the actual data of an EDC device before data conversion;
FIG. 13 is a view illustrating an example of the actual data of a Stat device after the data conversion;
FIG. 14 is a block diagram illustrating an example of a configuration of an information processing system of a second embodiment;
FIG. 15 is a view illustrating another example of an assignment screen;
FIG. 16 is a view illustrating an example of a case in which definition for collection and definition for statistics are generated of the second embodiment;
FIG. 17 is a flowchart illustrating an example of a definition generating process of the second embodiment; and
FIG. 18 is a view illustrating an example of a computer which executes a data format for clinical test creation supporting program.

### DESCRIPTION OF EMBODIMENTS

Preferred embodiments of the present invention will be explained with reference to accompanying drawings. Meanwhile, the disclosed technology is not limited by the embodiments. The following embodiments may be appropriately combined to the extent that they do not contradict each other.

### [a] First Embodiment

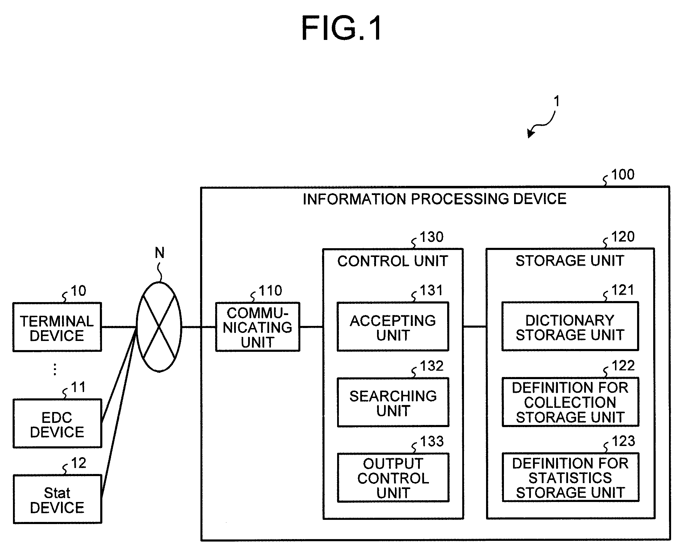

FIG. 1 is a block diagram illustrating an example of a configuration of an information processing system of a first embodiment. An information processing system 1 illustrated in FIG. 1 includes a terminal device 10, an EDC device 11, a Stat (statistics) device 12, and an information processing device 100. Meanwhile, although a case in which the system includes one terminal device 10 is illustrated in FIG. 1, the number of the terminal devices 10 is not limited and the information processing system 1 may also include any number of terminal devices 10. The EDC device 11 is an example of an EDC system and the Stat device 12 is an example of a CDISC-compliant statistical analysis system.

The terminal device 10, the EDC device 11, the Stat device 12, and the information processing device 100 are connected to one another such that they may communicate with one another through a network N. Any type of communication network both wired and wireless such as the Internet, a LAN (local area network), and a VPN (virtual private network) may be adopted as such network N.

The information processing device 100 obtains actual data of a clinical test from the EDC device 11, for example. The information processing device 100 accepts registration of a name of a data item used in the clinical test based on the obtained actual data. The information processing device 100 searches a first name associated with a second name corresponding to the accepted name of the data item with reference to a storage unit which stores the second name in association with the first name related to the data item used in the clinical test. Meanwhile, the first name is also represented as a standard name and the second name is also represented as an appellative in the following description. The information processing device 100 outputs a search result screen on which the first name searched from the storage unit is displayed. The information processing device 100 sets the first name selected on the search result screen as the name of the data item in the Stat device 12. According to this, it is possible to efficiently create a data format.

The terminal device 10 is a computer used by a user of the Stat device 12, for example. The terminal device 10 displays various screens and the like related to creation of a data format for clinical test received from the information processing device 100 to present to the user. The terminal device 10 may use a Web browser when displaying the various screens and the like and operating thereon, for example. The terminal device 10 transmits an obtaining instruction and various pieces of operation information to the information processing device 100. The terminal device 10 receives an assignment screen, the search result screen, and an assignment result screen from the information processing device 100 and displays the same on a display unit not illustrated. A portable personal computer may be adopted as an example of the terminal device 10. In addition to a portable terminal such as the above-described personal computer, a stationary personal computer may also be adopted as the terminal device 10. In addition to the above-described personal computer, a mobile communication terminal and the like such as a tablet terminal, a smartphone, a mobile phone, and a PHS (Personal Handyphone System) may be adopted, for example, as the portable terminal as the terminal device 10.

The EDC device 11 is a server which collects a case report form, that is to say, case data input by a doctor in a medical institution, for example. The EDC device 11 stores the collected case data in a database. The EDC device 11 may also have a function of a CDMS system which manages the case data. The EDC device 11 transmits the actual data of the case data stored in the database to the information processing device 100 based on a request from the information processing device 100. The EDC device 11 transmits the actual data of the case data stored in the database to the Stat device 12 based on a request from the Stat device 12.

The Stat device 12 is a server which generates CDISC-compliant clinical test data to be submitted to a regulatory authority, for example. The Stat device 12 converts the actual data of the case data received from the EDC device 11 to the CDISC-compliant clinical test data based on definition for statistics, that is to say, the data format received from the information processing device 100.

A configuration of the information processing device 100 is next described. As illustrated in FIG. 1, the information processing device 100 includes a communicating unit 110, a storage unit 120, and a control unit 130. Meanwhile, the information processing device 100 may also include various functional units included in the well-known computer, for example, the functional units such as various input devices and audio output devices in addition to the functional units illustrated in FIG. 1.

The communicating unit 110 is realized by a NIC (network interface card) and the like, for example. The communicating unit 110 is a communication interface connected to the terminal device 10, the EDC device 11, and the Stat device 12 by wire or without wire through the network N, the interface responsible for communication of information with the terminal device 10, the EDC device 11, and the Stat device 12. The communicating unit 110 receives the obtaining instruction and the operation information from the terminal device 10. The communicating unit 110 outputs the received obtaining instruction and operation information to the control unit 130. The communicating unit 110 transmits the assignment screen, the search result screen, and the assignment result screen input from the control unit 130 to the terminal device 10. The communicating unit 110 further receives the actual data of the case data from the EDC device 11. The communicating unit 110 outputs the received actual data of the case data to the control unit 130. The communicating unit 110 transmits the definition for statistics input from the control unit 130 to the Stat device 12.

The storage unit 120 is realized by a semiconductor memory device such as a RAM (random access memory) and a flash memory, and a storage device such as a hard disk and an optical disk, for example. The storage unit 120 includes a dictionary storage unit 121, a definition for collection storage unit 122, and a definition for statistics storage unit 123. The storage unit 120 also stores information used for processing by the control unit 130.

The dictionary storage unit 121 stores the appellative related to the data item of the database of the EDC device 11 in association with the standard name related to the data item of a CDISC-compliant database of the Stat device 12. The dictionary storage unit 121 includes a user dictionary which stores the standard name and the appellative in association with information for identifying the user and a system dictionary which stores the standard name and the appellative without association with the user, for example. That is to say, the user dictionary is a first storage unit and the system dictionary is a second storage unit. Meanwhile, since the user dictionary and the system dictionary have the same structure, the system dictionary is described as an example of the dictionary storage unit 121. The dictionary storage unit 121 includes a first table in which a domain, a column, and a dictionary name are stored and a second table in which the dictionary name, a term, and a synonym are stored in each of the user dictionary and the system dictionary.

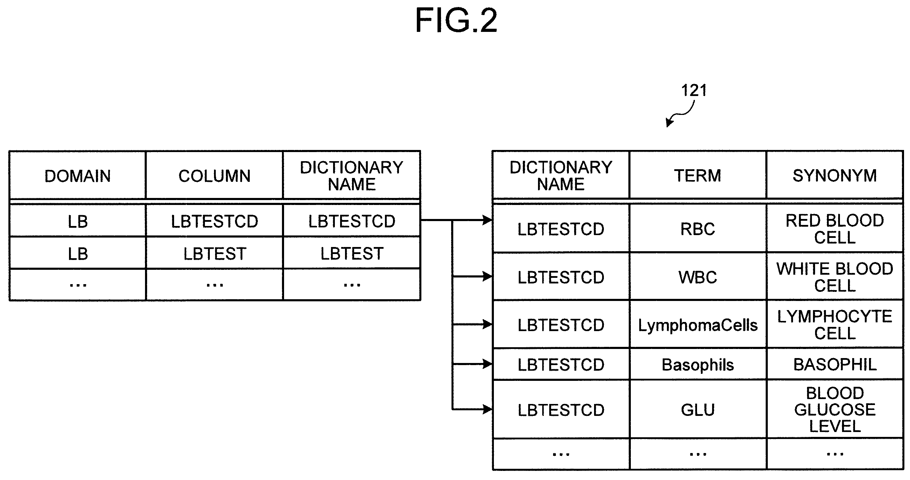

FIG. 2 is a view illustrating an example of the dictionary storage unit. As illustrated in FIG. 2, in the dictionary storage unit 121, the first table includes items such as "domain", "column", and "dictionary name", for example. In the dictionary storage unit 121, the second table includes items such as "dictionary name", "term", and "synonym", for example.

"Domain" in the first table is information indicating a broad category of the data item. The domain includes "LB" indicating a blood test, "VS" indicating a vital sign and the like, for example. "Column" is information indicating a column, that is to say, a variable of the database. The column includes "LBTESTCD" and the like for mapping the name of the data item, that is to say, an item label, for example. The column also includes "LBORRES" for mapping a numerical value, "LBORRESU" for mapping a unit and the like, for example, as another example not illustrated. "Dictionary name" is information specifying a dictionary name in the second table.

"Dictionary name" in the second table is information indicating the dictionary to which each term belongs. For example, when the dictionary name to be searched is specified, the terms belonging to the same dictionary are searched. Meanwhile, it may also be said that the dictionary name of the first table is a pointer and the dictionary name of the second table is a place indicated by the pointer. "Term" is information indicating the standard name entered in the column. That is to say, the term is a candidate displayed as an option of a value entered in the column. "Synonym" is information indicating the synonym corresponding to the term, that is to say, the appellative. The term and the synonym are in correspondence relationship such that "red blood cell" is the synonym of the term "RBC", for example.

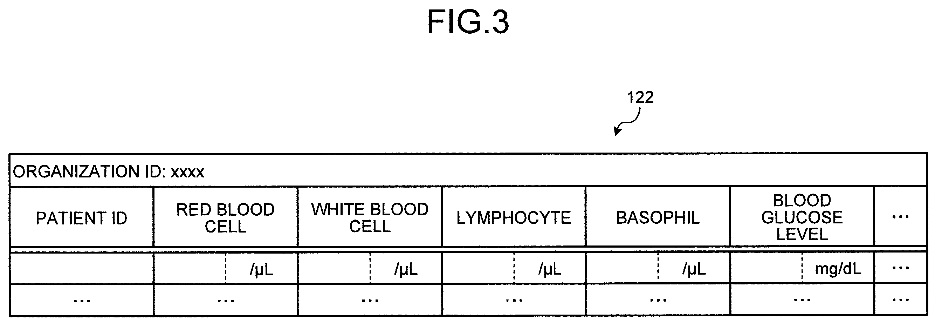

Returning to the description of FIG. 1, the definition for collection storage unit 122 stores definition for collection based on the name of the data item extracted from the actual data of the clinical test obtained from the EDC device 11 after the EDC device 11 collects the case data. Herein, the definition for collection is the data format corresponding to the EDC device 11. Meanwhile, the definition for collection based on the name of the data item extracted from the actual data is used for displaying the name of the data item on the assignment screen. It is also possible that the definition for collection is not stored when the data item is directly displayed on the assignment screen based on the name of the data item extracted from the actual data. FIG. 3 is a view illustrating an example of the definition for collection storage unit. As illustrated in FIG. 3, the definition for collection storage unit 122 includes items such as "organization ID", "patient ID", "red blood cell", "white blood cell", "lymphocyte", "basophil", and "blood glucose level". Meanwhile, although there are a row of items and a row of units corresponding to the items in the example in FIG. 3, there may also be another row.

"Organization ID" is an identifier for identifying an organization in which the clinical test is performed. "Patient ID" is an identifier for identifying a patient. "Red blood cell", "white blood cell", "lymphocyte", "basophil", and "blood glucose level" are information indicating examples of the name of the data item, that is to say, the item label and corresponding units. In the example in FIG. 3, the definition for collection defines the items and units of "red blood cell", "white blood cell", "lymphocyte", "basophil", and "blood glucose level" of each patient.

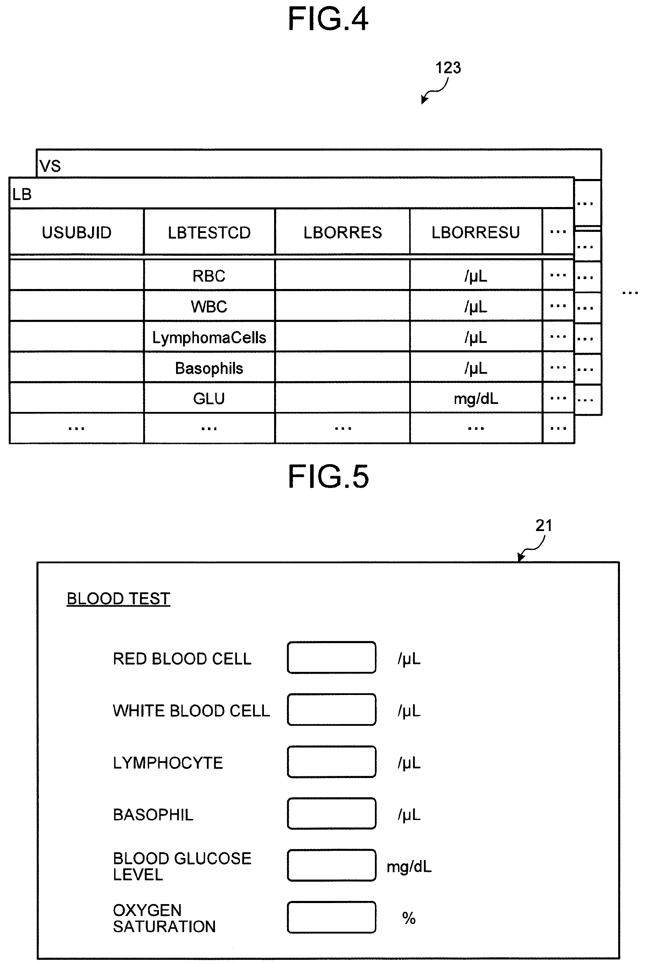

Returning to the description of FIG. 1, the definition for statistics storage unit 123 stores the definition for statistics, that is to say, the data format applied to the CDISC-compliant database of the Stat device 12. FIG. 4 is a view illustrating an example of the definition for statistics storage unit. As illustrated in FIG. 4, the definition for statistics storage unit 123 includes a plurality of tables corresponding to the domains. The definition for statistics storage unit 123 includes items such as "USUBJID", "LBTESTCD", "LBORRES", and "LBORRESU" for the table of the domain "LB", for example.

"USUBJID" is the item to which a user ID in the actual data of the EDC device 11 is mapped, for example. "LBTESTCD" is the item to which the name of the data item, that is to say, the item label in the actual data of the EDC device 11 is mapped, for example. "LBORRES" is the item to which a value such as the numerical value of the red blood cell, for example, in the actual data of the EDC device 11 is mapped, for example. "LBORRESU" is the item to which the unit in the actual data of the EDC device 11 is mapped, for example.

Returning to the description of FIG. 1, the control unit 130 is realized by execution of a program stored in an internal storage device on a RAM as a work area by a CPU, an MPU (micro processing unit) and the like, for example. The control unit 130 may also be realized by an integrated circuit such as an ASIC (application specific integrated circuit) and a FPGA (field programmable gate array), for example. The control unit 130 includes an accepting unit 131, a searching unit 132, and an output control unit 133 and realizes or executes a function and an effect of information processing hereinafter described. Meanwhile, an internal configuration of the control unit 130 is not limited to the configuration illustrated in FIG. 1 and may be another configuration as long as this performs the information processing to be described later. The control unit 130 executes operation on various screens based on the operation information input from the communicating unit 110.

When the accepting unit 131 receives the obtaining instruction from the terminal device 10 through the network N and the communicating unit 110, this obtains the actual data of the case data from the EDC device 11. Herein, an example of an input screen of the actual data in the EDC device 11 is described with reference to FIG. 5. FIG. 5 is a view illustrating an example of a doctor input screen. As illustrated in FIG. 5, a doctor input screen 21 on which "red blood cell" and the like are provided as the items of the blood test, for example, and an input field which accepts an input value to each item and a unit are arranged is displayed on a terminal device used by the doctor in the medical institution. The doctor in the medical institution inputs a result of the blood test of a certain patient by using the doctor input screen 21, for example. The name, the input value, and the unit of each data item on the doctor input screen 21 are transmitted from the terminal device of the doctor to the EDC device 11 to be stored in the database. The accepting unit 131 obtains the actual data of the case data stored in the EDC device 11 through the network N and the communicating unit 110.

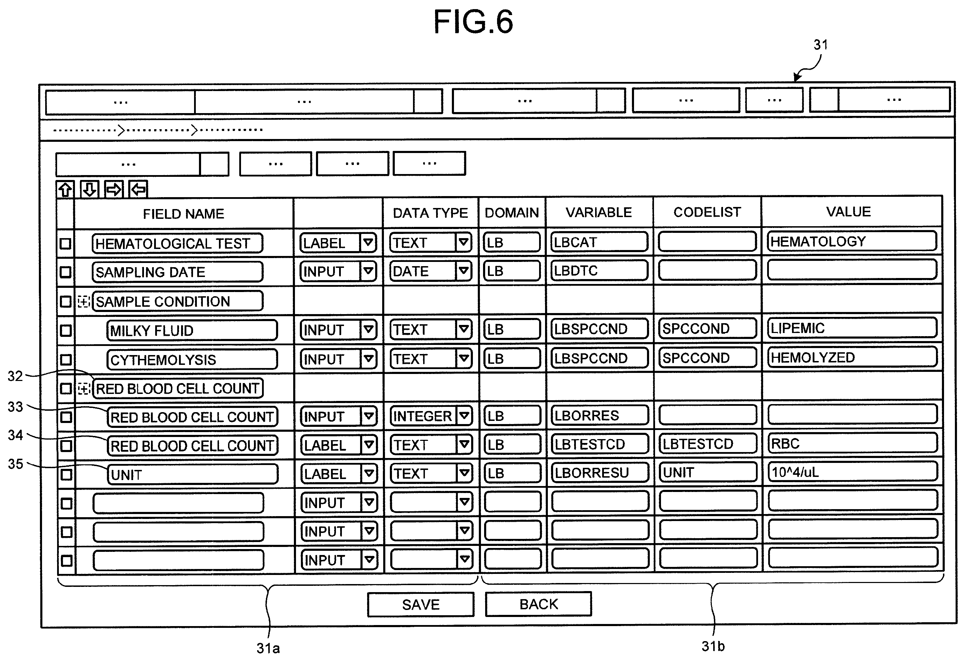

The accepting unit 131 generates the definition for collection based on the obtained actual data of the case data. That is to say, the accepting unit 131 accepts the registration of the name of the data item used in the clinical test. That is to say, the accepting unit 131 accepts the registration of the name of the data item extracted from the obtained actual data to generate the definition for collection as illustrated in FIG. 3, for example. The accepting unit 131 stores the generated definition for collection in the definition for collection storage unit 122. The accepting unit 131 generates the assignment screen based on the name of each data item of the definition for collection with reference to the definition for collection storage unit 122. The accepting unit 131 transmits the generated assignment screen to the terminal device 10 through the communicating unit 110 and the network N and displays the same on the display unit not illustrated. FIG. 6 is a view illustrating an example of the assignment screen. As illustrated in FIG. 6, an assignment screen 31 includes an area 31a in which the definition for collection generated from the actual data is displayed and an area 31b for generating the definition for statistics. Meanwhile, a state in which a value obtained from the system dictionary of the dictionary storage unit 121 is input as an initial value is displayed, for example, in the area 31b on the assignment screen 31.

The assignment screen 31 includes items such as "Field Name" indicating the name of the data item, that is to say, the item label and "Data Type" indicating a type of the value of the data item in the area 31a, for example. The assignment screen 31 also includes items such as "Domain" indicating the domain, "Variable" indicating the column (variable), "Codelist" indicating the dictionary name, and "Value" indicating the term in the area 31b, for example.

For example, "red blood cell count" of an item label 32 in the area 31a includes items such as "red blood cell count" of item labels 33 and 34 and "unit" of an item label 35 as lower-order items. The red blood cell count of the item label 33 corresponds to the domain and the column to which the value (type is numerical value) of the red blood cell count matches. That is to say, it is indicated that the value of the red blood cell count matches to the column "LBORRES" of the domain "LB" in the area 31b corresponding to the red blood cell count of the item label 33.

The red blood cell count of the item label 34 corresponds to the domain, the column, the dictionary name, and the term to which the item label (type is text) of the red blood cell count matches. That is to say, it is indicated that the term "RBC" included in the dictionary name "LBTESTCD" matches to the column "LBTESTCD" of the domain "LB" in the area 31b corresponding to the red blood cell count of the item label 34.

The unit of the item label 35 corresponds to the domain, the column, the dictionary name, and the term to which the unit (type is text) of the red blood cell count matches. That is to say, it is indicated that the term "10^4/uL" included in the dictionary name "UNIT" matches to the column "LBORRESU" of the domain "LB" in the area 31b corresponding to the unit of the item label 35.

When the item label, that is to say, the name of the data item displayed in the area 31a is selected, for example, on the assignment screen 31, the accepting unit 131 outputs the selected name of the data item to the searching unit 132. That is to say, when the accepting unit 131 receives the operation information indicating that the name of the data item is selected from the terminal device 10 through the network N and the communicating unit 110, this outputs the selected name of the data item to the searching unit 132.

Returning to the description of FIG. 1, when the selected name of the data item is input from the accepting unit 131, the searching unit 132 searches the standard name corresponding to the selected name of the data item with reference to the dictionary storage unit 121. In other words, the searching unit 132 searches the first name associated with the second name corresponding to the accepted name of the data item with reference to the storage unit which stores the second name in association with the first name related to the data item used in the clinical test.

The searching unit 132 outputs the search result screen generated based on a search result to the output control unit 133. The standard name associated with the appellative corresponding to the accepted name of the data item is displayed on the search result screen. The searching unit 132 sets the standard name selected by the user on the search result screen as the name of the data item of the definition for statistics. That is to say, the searching unit 132 maps the appellative to the standard name.

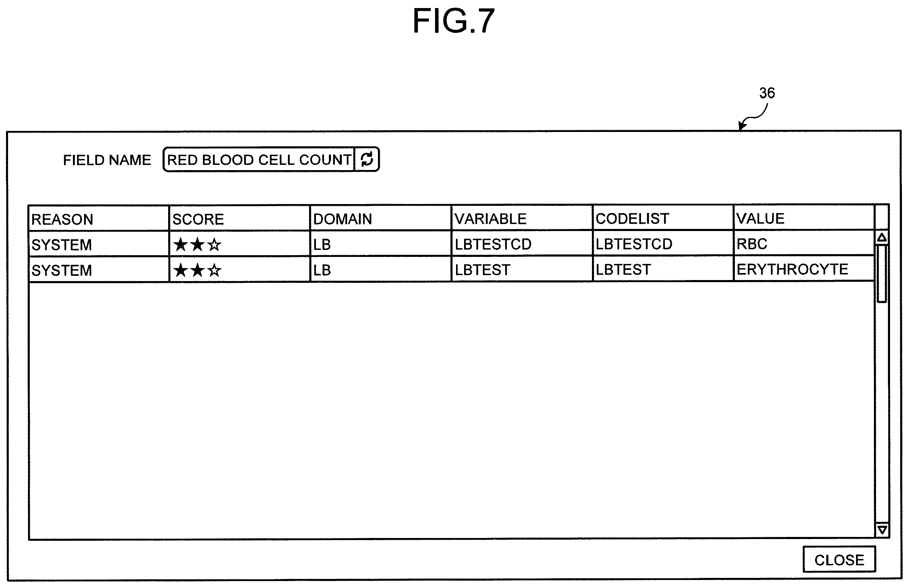

FIG. 7 is a view illustrating an example of the search result screen. As illustrated in FIG. 7, the standard name corresponding to "System" indicating search from the system dictionary of the dictionary storage unit 121 is displayed, for example, on a search result screen 36. On the search result screen 36, two candidates of the standard name in a case in which the appellative corresponding to the accepted name of the data item is "red blood cell count" are displayed, for example. In the example in FIG. 7, "RBC" corresponding to the column "LBTESTCD" and "Erythrocyte" corresponding to the column "LBTEST" are displayed as the terms corresponding to the red blood cell count.

Meanwhile, "Field Name" indicates the appellative, "Reason" indicates whether the dictionary is the system dictionary or the user dictionary, and "Score" indicates a matching degree between the appellative and the standard name by the number of starts on the search result screen 36. On the search result screen 36, "Domain" indicates the domain, "Variable" indicates the column, that is to say, the variable, "Codelist" indicates the dictionary name, and "Value" indicates the term. Meanwhile, in the following description, the similar representation is sometimes found in the screen example.

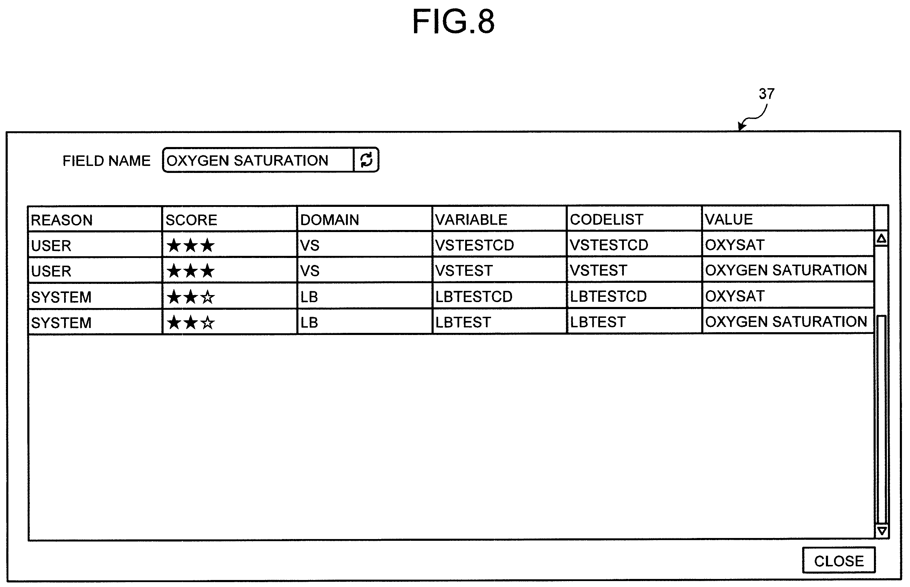

FIG. 8 is a view illustrating another example of the search result screen. A search result screen 37 illustrated in FIG. 8 is the example of a case in which the standard name associated with the appellative corresponding to the accepted name of the data item is in a plurality of domains. As illustrated in FIG. 8, the standard name corresponding to "User" indicating the search from the user dictionary of the dictionary storage unit 121 and the standard name corresponding to "System" indicating the search from the system dictionary of the dictionary storage unit 121 are displayed, for example, on the search result screen 37. On the search result screen 37, four candidates of the standard name in a case in which the appellative corresponding to the accepted name of the data item is "oxygen saturation" are displayed, for example. In the example in FIG. 8, "OXYSAT" corresponding to the domain "VS" and the column "VSTESTCD" of the user dictionary and "Oxygen Saturation" corresponding to the domain "VS" and the column "VSTEST" of the user dictionary are displayed as the terms corresponding to the oxygen saturation. "OXYSAT" corresponding to the domain "LB" and the column "LSTESTCD" of the system dictionary and "Oxygen Saturation" corresponding to the domain "LB" and the column "LBTEST" of the system dictionary are displayed as the terms corresponding to the oxygen saturation.

The searching unit 132 determines whether the setting for the name of each data item of the definition for collection is completed. The searching unit 132 waits for the selection of the name of the data item by the user on the assignment screen when the setting for the name of each data item is not completed. When the setting for the name of each data item is completed, the searching unit 132 stores the definition for statistics the setting of which is completed in the definition for statistics storage unit 123. The searching unit 132 generates the assignment result screen with reference to the definition for statistics storage unit 123 and outputs the generated assignment result screen to the output control unit 133.

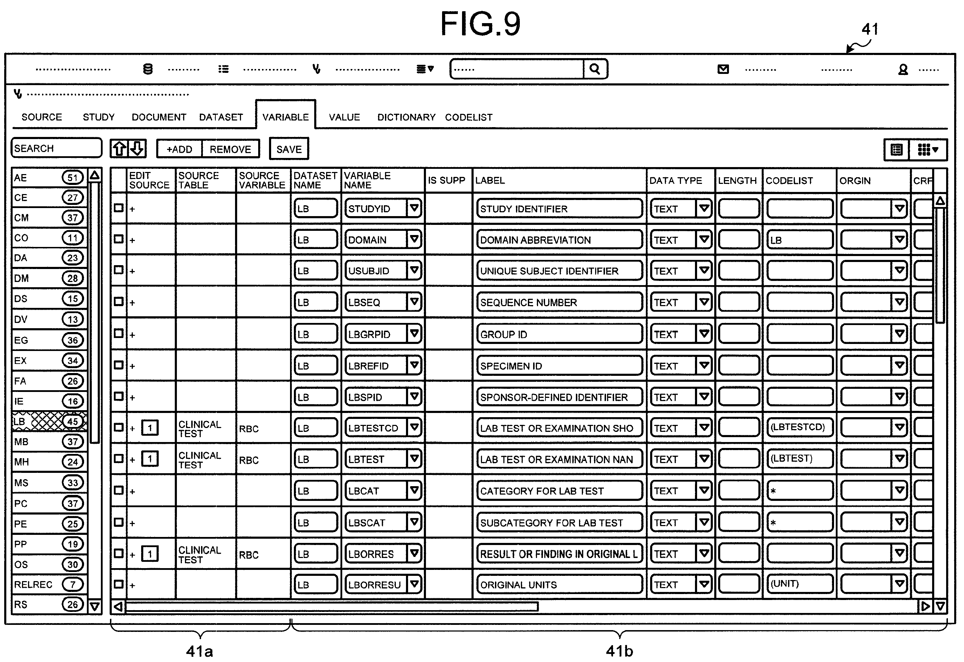

FIG. 9 is a view illustrating an example of the assignment result screen. As illustrated in FIG. 9, an assignment result screen 41 includes an area 41a and an area 41b. The area 41a displays the definition for collection corresponding to the definition for statistics, that is to say, a map source of the mapping. The area 41b displays the definition for statistics applied to the Stat device 12, that is to say, the data format in which the mapping of the name of the data item is completed. Meanwhile, FIG. 9 is an example of the assignment result screen about a part related to the red blood cell.

Returning to the description of FIG. 1, when the search result screen is input from the searching unit 132, the output control unit 133 transmits the input search result screen to the terminal device 10 through the communicating unit 110 and the network N and displays the same on the display unit not illustrated. When the assignment result screen is input from the searching unit 132, the output control unit 133 transmits the input assignment result screen to the terminal device 10 through the communicating unit 110 and the network N and displays the same on the display unit not illustrated.

The output control unit 133 accepts the operation to apply the definition for statistics corresponding to the displayed assignment result screen to the Stat device 12 on the assignment result screen. When the output control unit 133 accepts the operation, this transmits the definition for statistics to the Stat device 12 through the communicating unit 110 and the network N with reference to the definition for statistics storage unit 123.

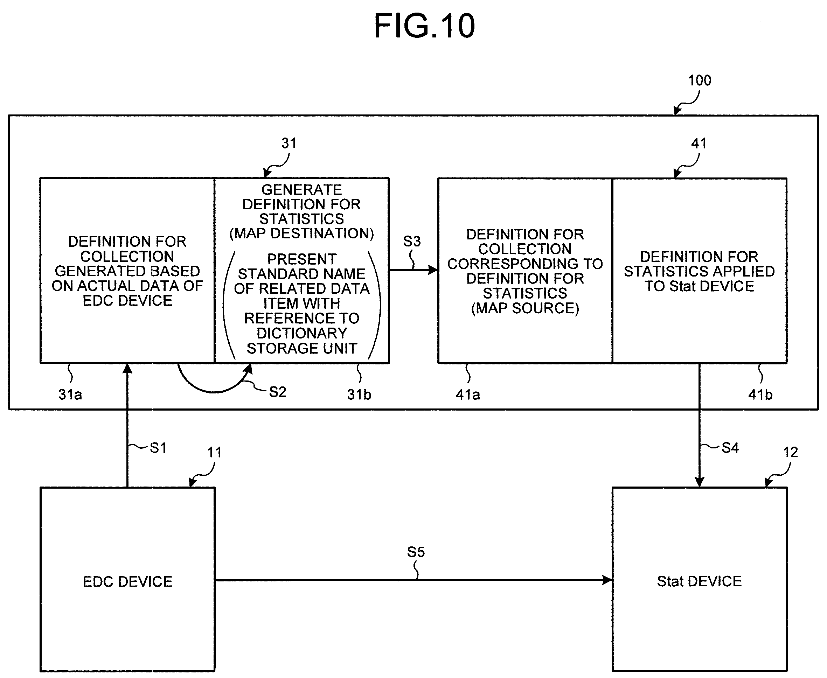

Next, the operation of the information processing system 1 of the first embodiment is described. First, a flow of generating the definition from the actual data of the EDC device 11 and apply the same to the Stat device 12, thereby performing data conversion is described. FIG. 10 is a view illustrating an example of a case in which the definition is generated from the actual data of the first embodiment.

When the receiving unit 131 of the information processing device 100 receives the obtaining instruction from the terminal device 10, this transmits a request to obtain the actual data to the EDC device 11. The EDC device 11 transmits the actual data of the case data stored in the database to the information processing device 100 based on a request from the information processing device 100. The receiving unit 131 of the information processing device 100 receives the actual data of the case data from the EDC device 11 to obtain (step S1).

The receiving unit 131 of the information processing device 100 generates the definition for collection based on the obtained actual data. The accepting unit 131 generates the assignment screen 31 based on the definition for collection. The assignment screen 31 includes the area 31a in which the definition for collection generated based on the actual data of the EDC device 11 is displayed and the area 31b for generating the definition for statistics. On the assignment screen 31, one or more dictionaries of the system dictionary and the user dictionary are referred to and the standard mane of the data item related to the name of the data item of the definition for collection is presented to the user. On the assignment screen 31, the name of the data item of the definition for statistics is set based on the selection by the user. That is to say, the definition for statistics indicating a map destination is generated on the assignment screen 31 (step S2).

The searching unit 132 of the information processing device 100 generates the assignment result screen 41 based on the definition for statistics the setting of which is completed when the setting for the name of each data item is completed (step S3). The assignment result screen 41 includes the area 41a in which the definition for collection corresponding to the definition for statistics indicating the map source is displayed and the area 41b in which the definition for statistics applied to the Stat device 12 is displayed. When the output control unit 133 accepts the operation to apply the displayed definition for statistics to the Stat device 12 on the assignment result screen 41, this transmits the definition for statistics to the Stat device 12 (step S4).

When the Stat device 12 receives the definition for statistics from the information processing device 100, this requests the EDC device 11 to transmit the actual data of the case data. The EDC device 11 transmits the actual data of the case data stored in the database to the Stat device 12 based on the request from the Stat device 12 (step S5). When the Stat device 12 receives the actual data of the case data, this converts the actual data of the case data to the CDISC-compliant clinical test data based on the definition for statistics. According to this, the information processing system 1 may efficiently create the data format and generate the CDISC-compliant clinical test data based on the created data format.

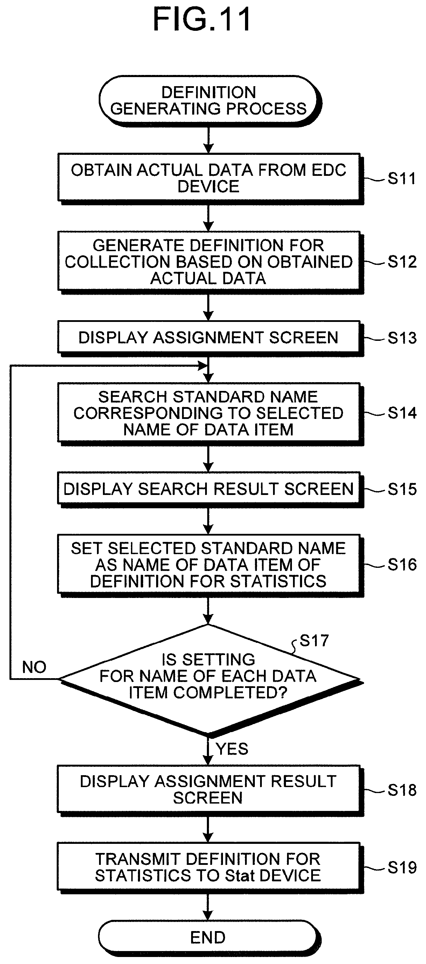

Subsequently, a definition generating process by the information processing device 100 is described. FIG. 11 is a flowchart illustrating an example of the definition generating process of the first embodiment.

When the accepting unit 131 of the information processing device 100 receives the obtaining instruction from the terminal device 10, this obtains the actual data of the case data from the EDC device 11 (step S11). The accepting unit 131 generates the definition for collection based on the obtained actual data (step S12). The accepting unit 131 stores the generated definition for collection in the definition for collection storage unit 122. The accepting unit 131 generates the assignment screen based on the name of each data item of the definition for collection with reference to the definition for collection storage unit 122. The accepting unit 131 transmits the generated assignment screen to the terminal device 10 and displays the assignment screen on the display unit not illustrated (step S13).

When the name of the data item of the definition for collection is selected on the assignment screen, the accepting unit 131 outputs the selected name of the data item to the searching unit 132. When the selected name of the data item is input from the accepting unit 131, the searching unit 132 searches the standard name corresponding to the selected name of the data item with reference to the dictionary storage unit 121 (step S14). The searching unit 132 outputs the search result screen generated based on a search result to the output control unit 133. When the search result screen is input from the searching unit 132, the output control unit 133 transmits the input search result screen to the terminal device 10 to display (step S15).

The searching unit 132 sets the standard name selected by the user on the search result screen as the name of the data item of the definition for statistics (step S16). The searching unit 132 determines whether the setting for the name of each data item of the definition for collection is completed (step S17). When the setting for the name of each data item is not completed (No at step S17), the searching unit 132 returns to step S14. When the setting for the name of each data item is completed (Yes at step S17), the searching unit 132 stores the definition for statistics the setting of which is completed in the definition for statistics storage unit 123. The searching unit 132 generates the assignment result screen with reference to the definition for statistics storage unit 123 and outputs the generated assignment result screen to the output control unit 133. When the assignment result screen is input from the searching unit 132, the output control unit 133 transmits the input assignment result screen to the terminal device 10 to display (step S18).

When the output control unit 133 accepts the operation to apply the definition for statistics corresponding to the displayed assignment result screen to the Stat device 12 on the assignment result screen, this transmits the definition for statistics to the Stat device 12 with reference to the definition for statistics storage unit 123 (step S19). According to this, the information processing device 100 may efficiently create the data format.

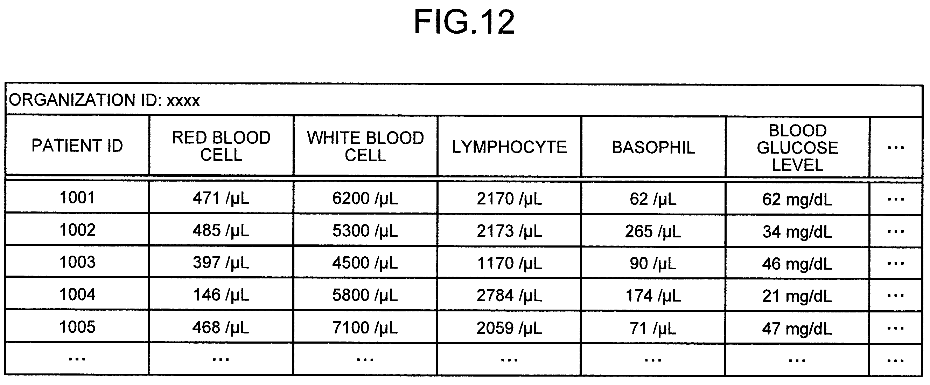

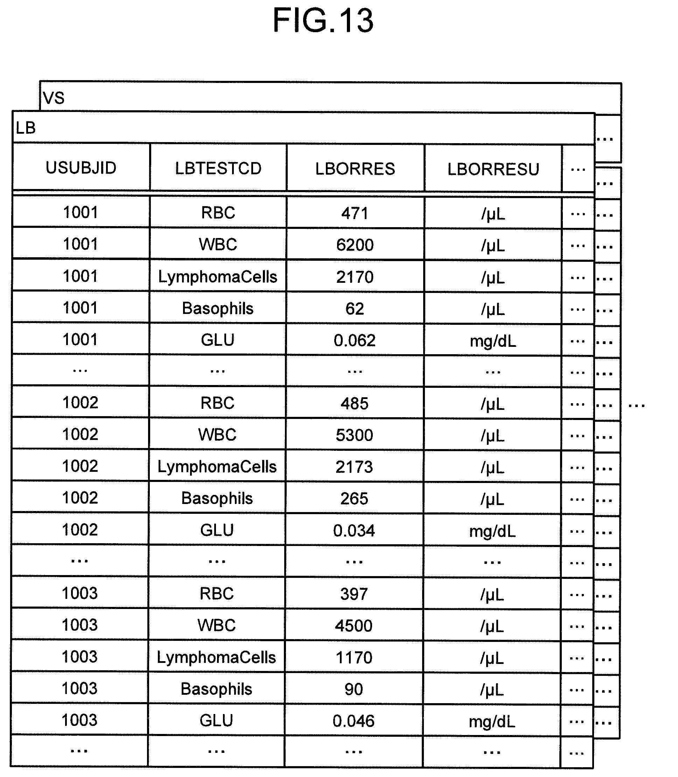

Herein, an example of the actual data before and after the data conversion is described with reference to FIGS. 12 and 13. FIG. 12 is a view illustrating the example of the actual data of the EDC device before the data conversion. As illustrated in FIG. 12, the actual data of the EDC device 11 before the data conversion, that is to say, the actual data of the case data includes items such as "patient ID", "red blood cell", "white blood cell", "lymphocyte", "basophil", and "blood glucose level" as columns for each organization ID indicating the organization in which the clinical test is performed, for example. The actual data of the case data is stored as one record for each patient ID, for example.

FIG. 13 is a view illustrating the example of the actual data of the Stat device after the data conversion. As illustrated in FIG. 13, the actual data of the Stat device 12 after the data conversion, that is to say, the CDISC-compliant clinical test data includes tables such as "LB" and "VS" for each domain, for example. Items such as "USUBJID", "LBTESTCD", "LBORRES", and "LBORRESU" are included in the "LB" table as columns, for example. The CDISC-compliant clinical test data is stored in association with the value and the unit of the item for each type of "LBTESTCD" indicating the test item, for example. That is to say, the data stored in a row direction in the actual data of the EDC device 11 is converted to be stored in a column direction in the actual data of the Stat device 12.

The information processing device 100 accepts the registration of the name of the data item used in the clinical test in this manner. The information processing device 100 searches the first name associated with the second name corresponding to the accepted name of the data item with reference to the storage unit which stores the second name in association with the first name related to the data item used in the clinical test. The information processing device 100 outputs the search result screen on which the first name searched from the storage unit is displayed. As a result, it is possible to efficiently create the data format.

The information processing device 100 searches the first name with reference to the first name and the second name corresponding to the user who registers the name from the storage unit which stores the first name and the second name in association with the information for identifying the user. As a result, the name of the data item after the conversion may be standardized in the organization to which the user belongs as long as this is registered in the user dictionary even though the name of the data item is not included in CDISC.

The information processing device 100 searches the first name with reference to the first name and the second name corresponding to the user who registers the name from the first storage unit which stores the first name and the second name in association with the information for identifying the user. The information processing device 100 searches the first name with reference to the second storage unit which stores the first name and the second name are stored without association with the user. As a result, the name of the data item after the conversion in the organization to which the user belongs may be selected from the name of the data item in the CDISC and the name of the data item registered in the user dictionary.

The information processing device 100 accepts the registration of the name of the data item based on the actual data of the clinical test. As a result, it is possible to efficiently create the data format used when the user of the Stat device 12 generates the clinical test data.

Although the definition for statistics is generated based on the actual data of the EDC device 11 in the above-described first embodiment, there is no limitation. For example, it is possible to reduce a processing cost at the time of statistical analysis after the clinical test if the definition for collection applied to the database of the EDC device 11 is generated to be applied before the clinical test starts. The embodiment of this case is hereinafter described as a second embodiment.

### [b] Second Embodiment

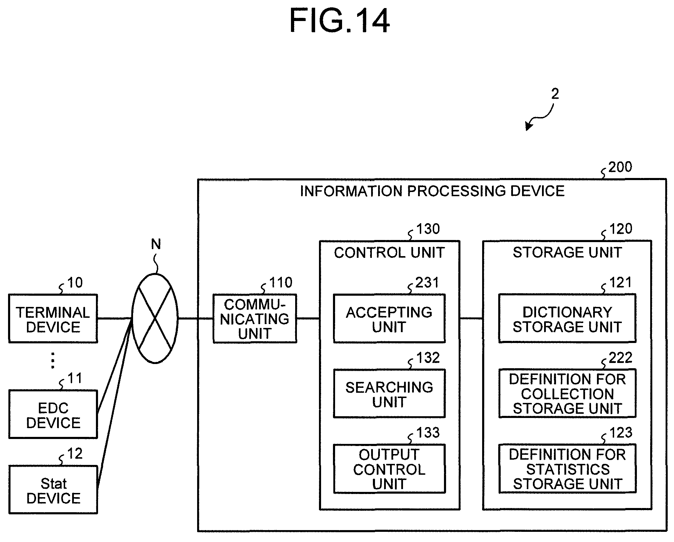

FIG. 14 is a block diagram illustrating an example of a configuration of an information processing system of a second embodiment. Meanwhile, the same reference numeral is assigned to the same configuration as that of the information processing system 1 of the first embodiment and the description of the configuration and operation is not repeated. An information processing system 2 of the second embodiment is different from the information processing system 1 of the first embodiment in that a name of a data item, that is to say, an item label is input by a user of an EDC device 11 in place of actual data of the EDC device 11.

The information processing system 2 of the second embodiment includes a terminal device 10, the EDC device 11, a Stat device 12, and an information processing device 200. The terminal device 10, the EDC device 11, the Stat device 12, and the information processing device 200 are connected to one another such that they may communicate with one another through a network N.

Although the user of the Stat device 12 uses the terminal device 10 in the first embodiment, the second embodiment is different in that the user of the EDC device 11 uses the same to transmit an input instruction in place of an obtaining instruction. The information processing device 200 is different from the information processing device 100 in including an accepting unit 231 and a definition for collection storage unit 222 in place of the accepting unit 131 and the definition for collection storage unit 122.

The definition for collection storage unit 222 of the information processing device 200 stores definition for collection based on the name of the data item input by the user of the EDC device 11 when constructing a database before case data is collected by the EDC device 11. Meanwhile, a structure of the definition for collection storage unit 222 is similar to that of the definition for collection storage unit 122, so that the description thereof is not repeated.

When the accepting unit 231 receives the input instruction of the item label from the terminal device 10 through the network N and a communicating unit 110, this generates an assignment screen. The accepting unit 231 transmits the generated assignment screen to the terminal device 10 through the communicating unit 110 and the network N and displays the same on a display unit not illustrated.

The accepting unit 231 accepts registration of the name of the data item used in a clinical test, that is to say, input of the item label of the definition for collection on the assignment screen. The accepting unit 231 outputs the input name of the data item to a searching unit 132. The accepting unit 231 also stores the input name of the data item in the definition for collection storage unit 222.

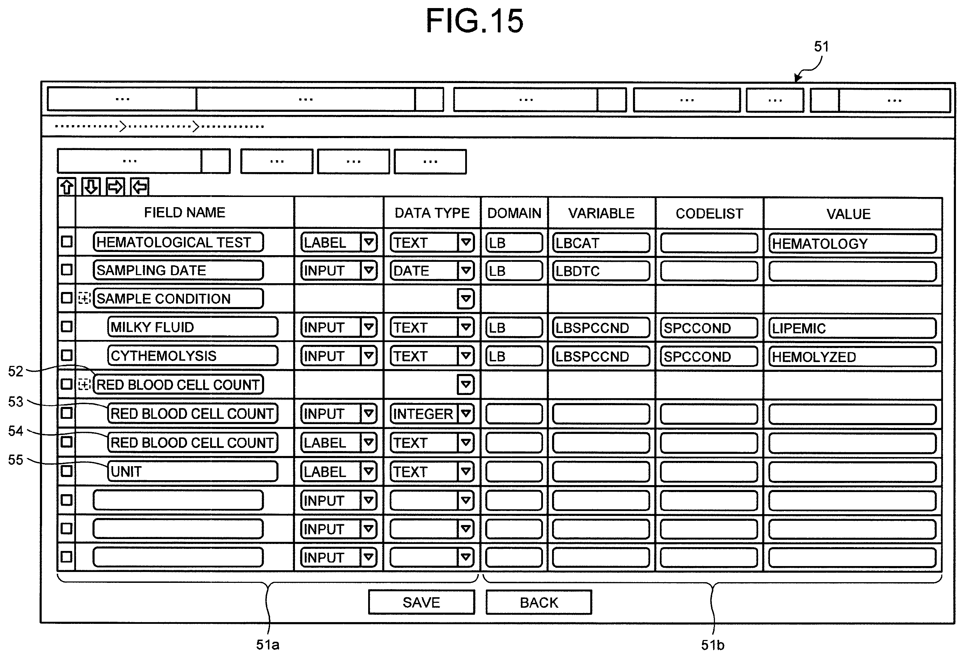

FIG. 15 is a view illustrating another example of the assignment screen. As illustrated in FIG. 15, an assignment screen 51 includes an area 51a in which the definition for collection based on the name of the data item input by the user of the EDC device 11 is displayed and an area 51b for generating definition for statistics. Meanwhile, a state in which setting is completed as far as the name of the data item "cythemolysis" in the area 51b of the assignment screen 51 is illustrated.

The assignment screen 51 includes items such as "Field Name" indicating the name of the data item, that is to say, the item label and "Data Type" indicating a type of a value of the data item in the area 51a, for example, as is the case with the assignment screen 31 of the first embodiment. The assignment screen 51 includes items such as "Domain" indicating a domain, "Variable" indicating a column (variable), "Codelist" indicating a dictionary name, and "Value" indicating a term in the area 51b, for example.

For example, "red blood cell count" of an item label 52 in the area 51a includes items such as "red blood cell count" of item labels 53 and 54 and "unit" of an item label 55 as lower-order items. The red blood cell count of the item label 53 corresponds to the domain and the column to which a value (type is numerical value) of the red blood cell count matches. The red blood cell count of the item label 54 corresponds to the domain, the column, the dictionary name, and the term to which the item label (type is text) of the red blood cell count matches. The unit of the item label 55 corresponds to the domain, the column, the dictionary name, and the term to which the unit (type is text) of the red blood cell count matches. On the assignment screen 51, an empty field in the domain, the column, the dictionary name, and the term indicates that a standard name is not yet selected. On the assignment screen 51, the name of the data item is set in the empty field in the area 51b when the standard name is selected on a search result screen by the user. That is to say, the assignment screen 51 is a screen for input on which the name of the data item is input.

When the setting for the name of each data item is completed by the searching unit 132, the accepting unit 231 transmits the definition for collection to the EDC device 11 through the communicating unit 110 and the network N with reference to the definition for collection storage unit 222.

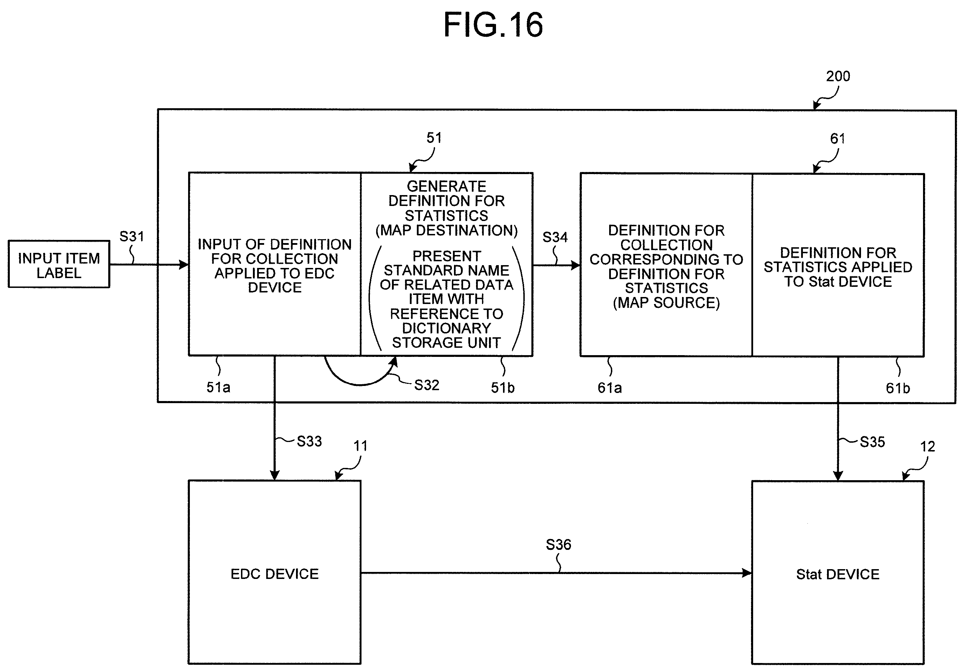

Next, operation of the information processing system 2 of the second embodiment is described. First, a flow of accepting the input of the name of the data item from the user of the EDC device 11 to generate the definition for collection and the definition for statistics and applying the same to the EDC device 11 and the Stat device 12 to perform data conversion is described. FIG. 16 is a view illustrating an example of a case in which the definition for collection and the definition for statistics are generated of the second embodiment.

When the accepting unit 231 of the information processing device 200 receives the input instruction of the item label from the terminal device 10, this generates the assignment screen 51 (step S31). The accepting unit 231 accepts the registration of the name of the data item used in the clinical test, that is to say, the input of the item label of the definition for collection on the assignment screen 51. The assignment screen 51 includes the area 51a in which the definition for collection generated based on the input by the user is displayed and the area 51b for generating the definition for statistics. On the assignment screen 51, the standard name of the data item related to the name of the data item of the definition for collection is presented to the user with reference to one or more dictionaries of a system dictionary and a user dictionary. On the assignment screen 51, the name of the data item of the definition for statistics is set based on the selection by the user. That is to say, the definition for statistics indicating a map destination is generated on the assignment screen 51 (step S32).

When the setting for the name of each data item is completed, the accepting unit 231 of the information processing device 200 transmits the definition for collection to the EDC device 11 with reference to the definition for collection storage unit 222 (step S33). When the EDC device 11 receives the definition for collection, this constructs the database based on the received definition for collection. Thereafter, in the EDC device 11, the case data is accumulated by doctors and the like.

When the setting for the name of each data item is completed, the searching unit 132 of the information processing device 200 generates an assignment result screen 61 based on the definition for statistics the setting of which is completed (step S34). The assignment result screen 61 includes an area 61a in which the definition for collection corresponding to the definition for statistics indicating a map source is displayed and an area 61b in which the definition for statistics applied to the Stat device 12 is displayed. When an output control unit 133 accepts operation to apply the displayed definition for statistics to the Stat device 12 on the assignment result screen 61, this transmits the definition for statistics to the Stat device 12 (step S35).

When the Stat device 12 receives the definition for statistics from the information processing device 200, this requests the EDC device 11 to transmit the actual data of the case data if the accumulation of the case data in the EDC device 11 is finished. The EDC device 11 transmits the actual data of the case data stored in the database to the Stat device 12 based on the request from the Stat device 12 (step S36). When the Stat device 12 receives the actual data of the case data, this converts the actual data of the case data to the CDISC-compliant clinical test data based on the definition for statistics. According to this, the information processing system 2 may efficiently create a data format, accumulate the case data in the EDC device 11 based on the created data format, and easily generate the CDISC-compliant clinical test data by the Stat device 12.

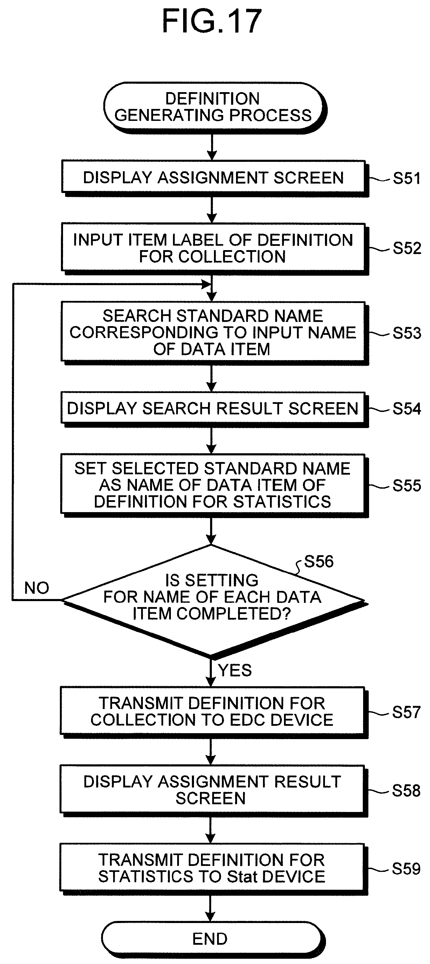

Subsequently, a definition generating process in the information processing device 200 is described. FIG. 17 is a flowchart illustrating an example of the definition generating process of the second embodiment.

When the accepting unit 231 of the information processing device 200 receives the input instruction of the item label from the terminal device 10, this generates the assignment screen. The accepting unit 231 transmits the generated assignment screen to the terminal device 10 and displays the same on the display unit not illustrated (step S51). The accepting unit 231 accepts the registration of the name of the data item used in the clinical test, that is to say, the input of the item label of the definition for collection on the assignment screen (step S52). The accepting unit 231 outputs the input name of the data item to a searching unit 132. The accepting unit 231 also stores the input name of the data item in the definition for collection storage unit 222.

When the name of the data item is input from the accepting unit 231, the searching unit 132 searches the standard name corresponding to the input name of the data item with reference to a dictionary storage unit 121 (step S53). The searching unit 132 outputs the search result screen generated based on a search result to the output control unit 133. When the search result screen is input from the searching unit 132, the output control unit 133 transmits the input search result screen to the terminal device 10 to display (step S54).

The searching unit 132 sets the standard name selected by the user on the search result screen as the name of the data item of the definition for statistics (step S55). The searching unit 132 determines whether the setting for the name of each data item of the definition for collection is completed (step S56). When the setting for the name of each data item is not completed (No at step S56), the searching unit 132 returns to step S53. When the setting for the name of each data item is completed (Yes at step S56), the searching unit 132 stores the definition for statistics the setting of which is completed in a definition for statistics storage unit 123.

The accepting unit 231 transmits the definition for collection to the EDC device 11 with reference to the definition for collection storage unit 222 (step S57). The searching unit 132 generates the assignment result screen with reference to the definition for statistics storage unit 123 and outputs the generated assignment result screen to the output control unit 133. When the assignment result screen is input from the searching unit 132, the output control unit 133 transmits the input assignment result screen to the terminal device 10 to display (step S58).

When the output control unit 133 accepts the operation to apply the definition for statistics corresponding to the displayed assignment result screen to the Stat device 12 on the assignment result screen, this transmits the definition for statistics to the Stat device 12 with reference to the definition for statistics storage unit 123 (step S59). According to this, the information processing device 200 may efficiently create the data format.

In this manner, the information processing device 200 accepts the registration of the name of the data item based on the input of the name of the data item on the screen for input. As a result, it is possible to generate and apply the definition for collection applied to the database of the EDC device 11 before the clinical test starts, so that a processing cost at the time of statistical analysis after the clinical test may be reduced.

Meanwhile, although the definition for collection and the definition for statistics are represented in a tabular format in the above-described embodiments, there is no limitation. For example, they may be described by a language such as XML (extensible markup language).

Although the definition for collection is generated before the clinical test starts in the above-described second embodiment, there is no limitation. For example, when the CDISC is revised during an implementation period of the clinical test, the definition for collection may be updated to be applied to the EDC device 11.

Components of each unit illustrated does not have to be physically configured as illustrated. That is to say, a specific form of dispersion/integration of each unit is not limited to that illustrated and it is possible to functionally or physically disperse/integrate to configure all or a part of the same in any unit according to various loads and use conditions. For example, the searching unit 132 and the output control unit 133 may be integrated.

Furthermore, all or any part of various processing functions performed by each device may be executed on a CPU (or a micro computer such as an MPU and an MCU (micro controller unit). It goes without saying that all or any part of the various processing functions may be executed on a program analyzed/executed by the CPU (or micro computer such as MPU and MCU) or hardware by wired logic.

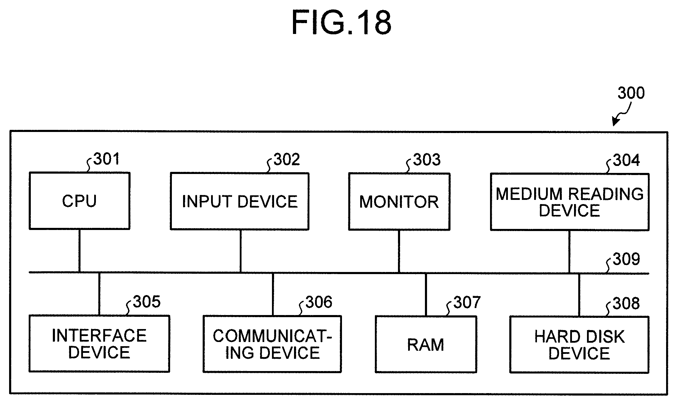

The various processes described in the above-described embodiments may be realized by execution of a program prepared in advance by a computer. Therefore, an example of the computer which executes the program having the function similar to that of the above-described embodiments is hereinafter described. FIG. 18 is a view illustrating an example of the computer which executes a data format for clinical test creation supporting program.

As illustrated in FIG. 18, a computer 300 includes a CPU 301 which executes various pieces of arithmetic processing, an input device 302 which accepts data input, and a monitor 303. The computer 300 also includes a medium reading device 304 which reads the program and the like from a storage medium, an interface device 305 for connecting to various devices, and a communicating device 306 for connecting to another information processing device and the like by wire or without wire. The computer 300 also includes a RAM 307 which temporarily stores various pieces of information and a hard disk device 308. The devices 301 to 308 are connected to a bus 309.

The data format for clinical test creation supporting program having the function similar to that of each processing unit of the accepting unit 131 or accepting unit 231, the searching unit 132, and the output control unit 133 illustrated in FIG. 1 or FIG. 14 is stored in the hard disk device 308. The dictionary storage unit 121, the definition for collection storage unit 122 or definition for collection storage unit 222, the definition for statistics storage unit 123, and various data for realizing the supporting program are stored in the hard disk device 308. The input device 302 accepts input of various pieces of information such as management information from a manager of the computer 300, for example. The monitor 303 displays various screens such as a screen of the management information for the manager of the computer 300, for example. A printing device and the like is connected, for example, to the interface device 305. The communicating device 306 having the function similar to that of the communicating unit 110 illustrated in FIG. 1 or FIG. 14 is connected to the network N, for example, to communicate various pieces of information with the terminal device 10, the EDC device 11, and the Stat device 12.

The CPU 301 reads each program stored in the hard disk device 308 and develops the same on the RAM 307 to execute, thereby performing various processes. The programs may allow the computer 300 to serve as the accepting unit 131 or accepting unit 231, the searching unit 132, and the output control unit 133 illustrated in FIG. 1 or FIG. 14.

Meanwhile, the above-described data format for clinical test creation supporting program does not have to be stored in the hard disk device 308. For example, the computer 300 may read to execute the program stored in the storage medium which the computer 300 may read. The storage medium which the computer 300 may read includes a portable recording medium such as a CD-ROM, a DVD disk, and a USB (universal serial bus) memory, the semiconductor memory such as the flash memory, a hard disk drive and the like, for example. It is also possible to store the supporting program in the device connected to a public line, the Internet, the LAN and the like such that the computer 300 reads the supporting program therefrom to execute.

The data format may be efficiently created.

## Claims

1. A data format for clinical test creation supporting program that causes a computer to execute process comprising:
accepting (131) registration of a name of a data item used in a clinical test;
searching (132) a first name associated with a second name corresponding to the accepted name of the data item with reference to a storage unit that stores the second name in association with the first name related to the data item used in the clinical test; and
outputting (133) a search result screen on which the first name searched from the storage unit is displayed.

2. The data format for clinical test creation supporting program according to claim 1, wherein the searching (132) includes searching the first name with reference to the first name and the second name corresponding to a user who registers the name from the storage unit that stores the first name and the second name in association with information for identifying the user.

3. The data format for clinical test creation supporting program according to claim 1, wherein the searching (132) includes searching the first name with reference to the first name and the second name corresponding to a user who registers the name from a first storage unit that stores the first name and the second name in association with information for identifying the user, and searching the first name with reference to a second storage unit that stores the first name and the second name without association with the user.

4. The data format for clinical test creation supporting program according to any one of claims 1 to 3, wherein the accepting (131) includes accepting the registration of the name of the data item based on actual data of the clinical test.

5. The data format for clinical test creation supporting program according to any one of claims 1 to 3, wherein the accepting (131) includes accepting the registration of the name of the data item based on input of the name of the data item on a screen for input.

6. A method for supporting creation of a data format for clinical test, the method comprising:
accepting (131) registration of a name of a data item used in a clinical test, using a processor;
searching (132) a first name associated with a second name corresponding to the accepted name of the data item with reference to a storage unit that stores the second name in association with the first name related to the data item used in the clinical test, using the processor; and
outputting (133) a search result screen on which the first name searched from the storage unit is displayed, using the processor.

7. The method according to claim 6, wherein the searching (132) includes searching the first name with reference to the first name and the second name corresponding to a user who registers the name from the storage unit that stores the first name and the second name in association with information for identifying the user.

8. The method according to claim 6, wherein the searching (132) includes searching the first name with reference to the first name and the second name corresponding to a user who registers the name from a first storage unit that stores the first name and the second name in association with information for identifying the user and searching the first name with reference to a second storage unit that stores the first name and the second name without association with the user.

9. The method according to any one of claims 6 to 8, wherein the accepting (131) includes accepting the registration of the name of the data item based on actual data of the clinical test.

10. The method according to any one of claims 6 to 8, wherein the accepting (131) includes accepting the registration of the name of the data item based on input of the name of the data item on a screen for input.

11. An information processing device (100), comprising:
an accepting unit (131) that accepts registration of a name of a data item used in a clinical test;
a searching unit (132) that searches a first name associated with a second name corresponding to the accepted name of the data item with reference to a storage unit that stores the second name in association with the first name related to the data item used in the clinical test; and
an output control unit (133) that outputs a search result screen on which the first name searched from the storage unit is displayed.

12. The information processing device according to claim 11, wherein the searching unit (132) searches the first name with reference to the first name and the second name corresponding to a user who registers the name from the storage unit that stores the first name and the second name in association with information for identifying the user.

13. The information processing device according to claim 11, wherein the searching unit (132) searches the first name with reference to the first name and the second name corresponding to a user who registers the name from a first storage unit that stores the first name and the second name in association with information for identifying the user, and searching the first name with reference to a second storage unit that stores the first name and the second name without association with the user.

14. The information processing device according to any one of claims 11 to 13, wherein the accepting unit (131) accepts the registration of the name of the data item based on actual data of the clinical test.

15. The information processing device according to any one of claims 11 to 13, wherein the accepting unit (131) accepts the registration of the name of the data item based on input of the name of the data item on a screen for input.
